# EUROPEAN PATENT APPLICATION

(11) **EP 0 692 225 A2**
(43) Date of publication of application: **17.01.1996**
(21) Application number: 95109137.0
(22) Date of filing: 13.06.1995
(51) Int. Cl.: A61F 2/00

(54) **Surgical support mesh**

(30) Priority: 15.06.1994 US 260116
(71) Applicant: Meadox Medicals, Inc., Oakland New Jersey 07436 (US)
(72) Inventor: Schmitt, Peter J., Garnerville, New York 10923 (US)
(74) Representative: Prechtel, Jörg, Dipl.-Phys. Dr.

(57) **Abstract**

A soft and pliable surgical support mesh exhibiting increased resistance to inhabitation of infectious matter. The mesh includes a support trellis formed of multifilament yarns wherein the interstitial voids located between the filaments of said yarns are enclosed within an infection-impervious matrix.

## Description

### BACKGROUND OF THE INVENTION

The present invention relates to surgical mesh and, more particularly, to a soft and pliable multifilament surgical support mesh exhibiting improved resistance to inhabitation of bacteria and other infectious matter.

Surgical mesh is typically used for repair and restoration of living tissue. For example, surgical mesh may be used to support and/or reinforce a damaged or weakened portion of the body. In this regard, the mesh must be sufficiently porous to allow for growth of tissue through the graft after implantation. The healing tissue grows through porous openings in the implanted mesh, thereby assimilating the mesh and adding structural integrity to the tissue.

Surgical mesh may be produced by knitting, weaving, braiding or otherwise forming a plurality of yarns into a support trellis. Moreover, such mesh may be produced with monofilament or multifilament yarns made of materials such as polypropylene and polyester. Surgical mesh formed of monofilament yarn provides satisfactory reinforcement ability, but is generally stiff and has limited pliability. In contrast, surgical mesh formed of multifilament yarn is soft and pliable in comparison to mesh formed of monofilament yarn.

However, mesh formed of multifilament yarn may tend to harbor infectious matter such as bacteria. Particularly, the small void areas or interstitial spaces between the filaments of a multifilament yarn may promote the breading of such bacteria. To date, surgeons typically prefer the monofilament design because of its improved resistance to harboring of infectious matter. As a result of this choice, surgeons must forego the advantages associated with multifilament yarns.

An example of a prior art surgical mesh is disclosed in U.S. Patent No. 2,671,444. The surgical mesh described therein is an integral network of interconnecting yarns formed by molding a polyethylene resin. In essence, the '444 mesh is a molded, monofilament mesh and, hence, is relatively stiff and exhibits limited pliability.

U.S. Patent No. 3,054,406 discloses another example of a surgical mesh used for repair and restoration of living tissue. The surgical mesh described therein may be woven from either monofilament or multifilament polyethylene yarns. The mesh has limited pliability when formed of monofilament yarns, and may be prone to harboring of infectious matter when formed of multifilament yarns.

U.S. Patent No. 4,452,245 discloses still another example of a surgical mesh. The surgical mesh described therein is formed with monofilament polypropylene yarns which are knitted into a continuous tubular shape. The knitted mesh is porous and exhibits infection-resistant characteristics because of its monofilament construction. However, the monofilament mesh tends to be stiff and relatively non-pliable, which detracts from the body's ability to incorporate the mesh.

There is therefore a need in the art for a surgical support mesh which exhibits both the soft and pliable characteristics of a mesh produced from multifilament yarns and the infection resistance of a mesh produced from monofilament yarns. The mesh should also be non-linting, fray resistant and ravel resistant.

### SUMMARY OF THE INVENTION

The present invention, which addresses the needs of the art, provides a soft and pliable surgical mesh. The mesh includes a support trellis formed of multifilament yarns encapsulated within an infection-impervious matrix whereby the interstitial voids located between the filaments of the yarns are enclosed within the matrix.

The present invention also provides a method of producing a soft and pliable surgical mesh exhibiting increased resistance to inhabitation of infectious matter from a support trellis formed of multifilament yarns. The method includes the step of encapsulating the multifilament yarns within an infection-impervious matrix whereby the interstitial voids located between the filaments of the yarns are enclosed within the matrix.

Finally, the present invention provides a method of repairing a damaged portion of a patient's body. The method includes the step of providing a surgical mesh. The mesh includes a support trellis formed of multifilament yarns encapsulated within an infection-impervious matrix whereby the mesh is soft and pliable while simultaneously exhibiting a resistance to inhabitation of infectious matter. The method includes the further step of accessing the damaged portion of the body. Finally, the method includes the step of implanting the surgical mesh in the body to reinforce the damaged portion and allowing the mesh to assimilate into the body.

As a result, the present invention provides a surgical support mesh which exhibits both the soft and pliable characteristics of a mesh produced from multifilament yarns and the infection resistance of a mesh produced from monofilament yarns. Moreover, the present invention provides a surgical support mesh which is non-linting, fray resistant and ravel resistant.

### BRIEF DESCRIPTION OF DRAWINGS

Fig. 1 shows a portion of a woven prior art support mesh made of multifilament yarns;
Fig. 1a is a sectional view taken along lines 1a-1a of Fig. 1;
Fig. 2 shows a portion of a support mesh of the present invention wherein a woven support trellis formed of multifilament yarns has been encapsulated within an infection-impervious matrix in accordance with the present invention;
Fig. 2a is a sectional view taken along lines 2a-2a of Fig. 2;
Fig. 3 is a sectional view of a multifilament yarn which has been encapsulated within an infection-impervious matrix prior to forming of the support trellis;
Fig. 4 is a view similar to Fig. 3, wherein the encapsulating resin which forms the matrix has penetrated into the yarn and has substantially filled the interstitial voids;
Fig. 5 is a sectional view of a multifilament yarn formed of bi-component fibers;
Fig. 6 is an enlarged sectional view of an individual bi-component fiber;
Fig. 7 shows a portion of a woven support mesh of the present invention formed of the bi-component multifilament yarns of Fig. 5 which have been fused following fabrication of the trellis; and
Fig. 7a is a sectional view taken along lines 7a-7a of Fig. 7.

### DETAILED DESCRIPTION OF THE INVENTION

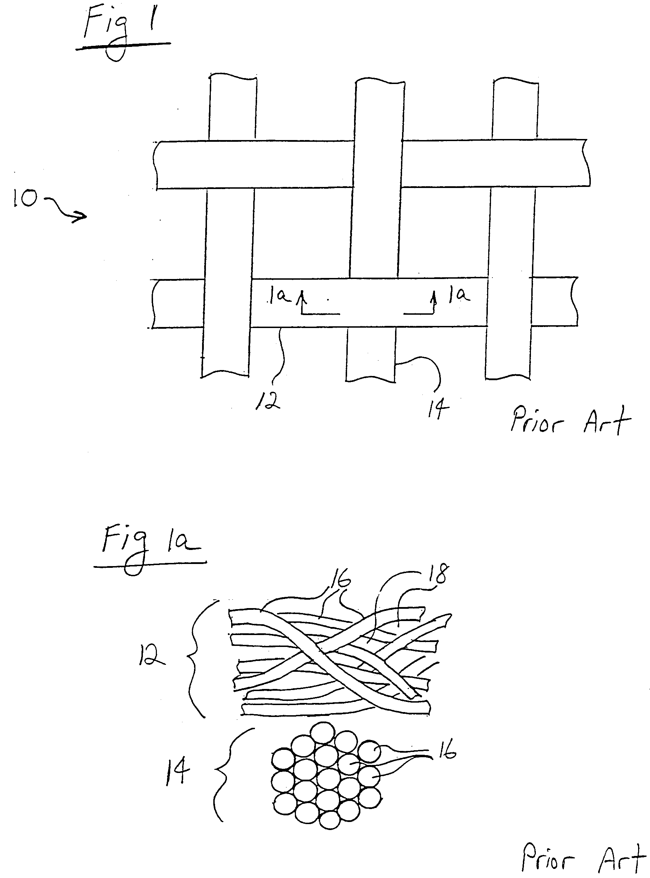

Referring to the drawings and, in particular to Fig. 1, therein illustrated is a prior art surgical support mesh 10. Mesh 10 may be manufactured from monofilament or multifilament yarns. Prior art mesh 10, as shown, includes multifilament horizontally-extending yarns 12 and multifilament vertically-extending yarns 14 woven together to form a support trellis.

The use of multifilament yarns, such as yarns 12 and 14, provides a mesh having greater pliability and suppleness than the use of monofilament yarns. These characteristics result from both the smaller diameter of the individual filaments and the interstitial spaces or voids that are located between such filaments.

In particular, the flexibility of a filament (or fiber) generally increases as its diameter decreases. Because the solid cross-sectional area of the filaments of a multifilament yarn is less than the cross-sectional area of a monofilament yarn of equivalent diameter, the multifilament yarn will have a greater degree of flexibility and pliability than that of the monofilament yarn.

As shown in Fig. 1a, each of multifilament yarns 12 and 14 is composed of a plurality of filaments 16 that are twisted together to form the yarn. Interstitial spaces 18, which are pockets of air, are formed between adjacent filaments of the yarn. Although these voids contribute to the softness and pliability of the formed mesh, they also provide a natural breeding ground for bacteria or other infectious material.

Surgical mesh is, of course, thoroughly sterilized prior to implantation. Nevertheless, surgeons typically prefer the use of monofilament-designed mesh to minimize any risk of infection. As a result, the advantages associated with multifilament-designed mesh (i.e., softness and pliability which result in better assimilation of the mesh into the body) are typically sacrificed.

It has been discovered herein that a surgical support mesh having both the softness and pliability of a multifilament-designed mesh and the infection resistance of a monofilament-designed mesh may be produced. Particularly, it has been discovered that a support trellis formed of multifilament yarn wherein the interstitial voids located between adjacent filaments are enclosed within an infection-impervious matrix exhibits the desired resistance to harboring of infectious matter without significant loss of flexibility.

Particularly, the matrix, which completely encloses the interstitial voids between the filaments of the yarn, provides an effective barrier to the passage of infectious matter between the interior and exterior of the yarn. Accordingly, any voids remaining in the yarn after encapsulation of such yarn are enclosed (and thereby sealed) within the resultant matrix.

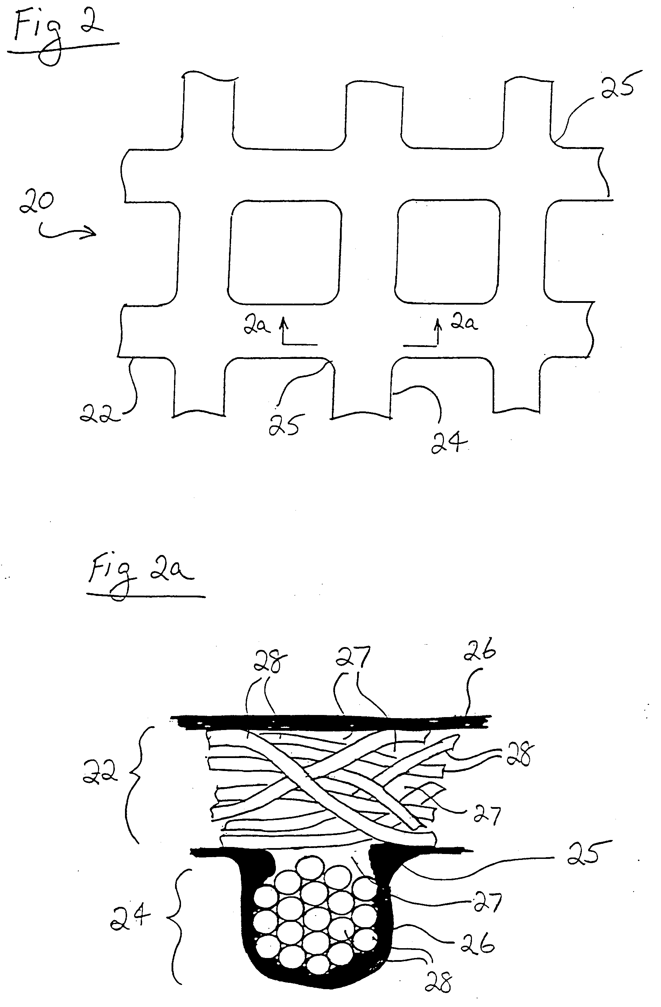

A first embodiment of the present invention is shown in Fig. 2. Particularly, this first embodiment includes a support trellis 20 formed of multifilament yarns 22 and 24 which overlap at cross-over junctions 25. Subsequent to forming of the trellis, such trellis is encapsulated within a matrix 26, which is preferably a flexible material that continuously surrounds the exterior of the yarns thereby enclosing interstitial voids 27 located between filaments 28 (see Fig. 2a). In one embodiment, the matrix is formed from a polymeric resin.

As shown in Fig. 2a, the resin can be applied to the yarn in such a manner as to not allow the resin to substantially penetrate into the yarn. Particularly, the penetration of the resin can be controlled through the application procedure, e.g., quantity of resin applied and/or encapsulating time. In such an embodiment, the interstitial spaces are enclosed (rather than filled) within the continuous matrix. However, it is contemplated that the resin can be allowed to penetrate into the yarn, thereby substantially filling the void space located therein.

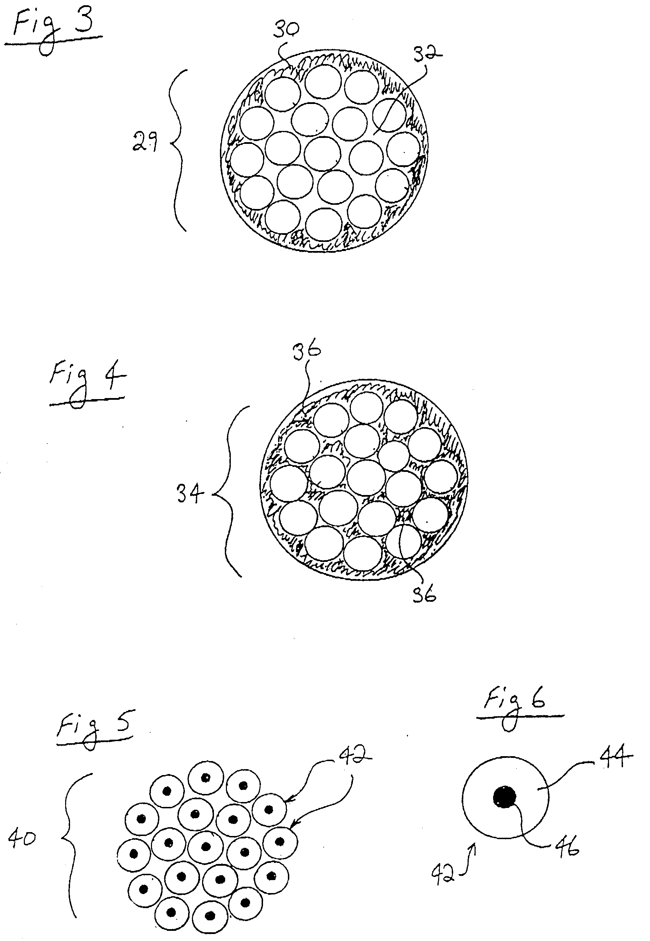

In another embodiment of the present invention, individual yarns 29, as shown in Fig. 3, are encapsulated within matrix 30 prior to forming of the support trellis. As a result, interstitial voids 32 remaining in the yarn are enclosed (and thereby sealed) within the matrix. This then prevents infectious matter from traveling between the interior and exterior of the yarn. Stated differently, the matrix provides an infection-impervious barrier between any interstitial voids remaining in the yarn after encapsulation and the exterior of such yarn, while simultaneously maintaining the desired flexibility.

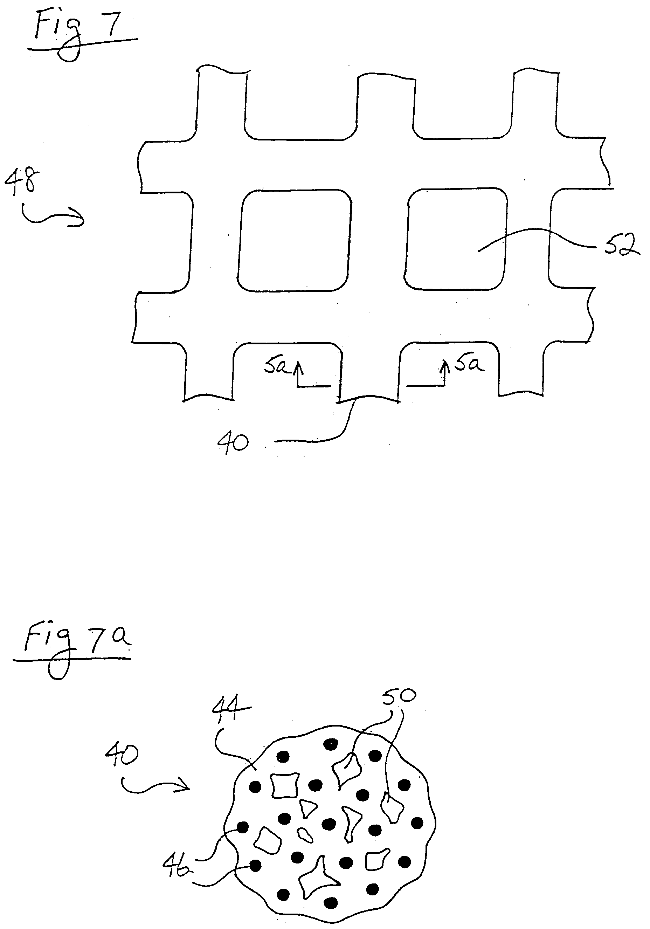

As mentioned, the depth of penetration of the matrix can be controlled by regulating the quantity of resin applied to the yarn and/or by regulating the coating time. For example, referring to yarn 34 shown in Fig. 4, matrix 36 penetrates into the interstitial spaces of the yarn, thereby substantially filling the bulk of air space located therein.

The resin employed to encapsulate the trellis preferably has a melting temperature lower than the melting temperature of the individual filaments such that the resin may be applied to the trellis without damage thereto (i.e., melting of the filaments). Moreover, the resin should exhibit a high degree of flexibility to ensure that the formed mesh retains its desired pliability. Preferably, the resin has a Young's Modulus lower than that of the filament material. Resins formed from polyester, polypropylene, polyethylene, polyurethane or copolymers thereof are contemplated for use herein.

In one embodiment of the present invention, a resin solution is applied to the formed trellis. The solvent carrying the resin is then caused to be evaporated, whereby the solute impregnates and thereby fills the voids within the yarn.

The encapsulation of the multifilament yarns permanently encloses the interstitial spaces formed between the individual filament of the yarns. Particularly, a continuous infection-impervious matrix is formed around the exterior of the yarn, thereby encapsulating the filaments and filling and/or sealing the interstitial spaces formed therebetween. The resultant surgical mesh therefore exhibits the softness and pliability of a multifilament yarn, while simultaneously providing a barrier to the passage of infectious matter.

Moreover, the encapsulation of the yarns, if done subsequent to forming of the support trellis fuses the yarns of the trellis together at the crossover junctions 25 shown in Fig. 2. This improves the ravel resistance of the formed mesh. It also improves the linting and fraying characteristics of the mesh (i.e., the mesh is less prone to both linting and fraying). If, however, the individual yarns are encapsulated prior to forming of the trellis, the trellis may still be heated after formation to fuse the yarn coatings together, thereby rendering such trellis ravel resistant.

Referring to Figs. 5 and 6, the multifilament yarns employed in the present invention, e.g., yarn 40, may be formed of bi-component filaments 42. Each of these bi-component filaments includes a sheath 44 and a core 46. The sheath is formed of a material having a melting or fusing point lower than that of the material forming the core of the filament. Thus, when the filament is heated to a particular temperature, the sheath will soften and flow together with sheaths from adjacent filaments, thereby filling the void space between filaments and encapsulating the cores of such filaments.

The sheath 44 is preferably a polyethylene terephthalate/isophthalate co-polyester, while the core 46 is preferably a polyethylene terephthalate polyester. Of course, other suitable materials may be used to manufacture the bi-component filaments.

Another embodiment of the present invention is shown in Figs. 7 and 7a. Particularly, this embodiment includes a support trellis 48 formed of bi-component multifilament yarns, such as yarns 40. Subsequent to forming of the trellis, the trellis is heated to a predetermined temperature, i.e., the fusing temperature of the bi-component filaments.

It is at this fusing temperature that sheaths 44 of the bi-component filament begin to melt and flow together, thereby at least substantially filling the voids between filaments and also encapsulating cores 46 within a continuous polymeric matrix. The melting sheaths also enclose any voids 50 which are not filled by the flowing polymer. Because the polymer of sheath 44 is softer and more flexible than the polymer of core 46, the formed trellis exhibits the flexibility of a surgical support mesh formed of a conventional multifilament yarn.

The surgical mesh of the present invention may be formed by weaving, knitting, braiding or otherwise forming a plurality of multifilament yarns into a support trellis structure. This multifilament yarns may be either traditional multifilament yarns or bi-component multifilament yarns. When formed of bi-component multifilament yarns, the support mesh may thereafter be radiated with thermal or light energy to fuse the filaments together. For example, the mesh may be placed in an oven and heated to a temperature of, for example, 180°C such the sheaths of the individual filaments fuse together.

The encapsulation of the yarns, whether by coating or fusing of bi-component filaments, provides the trellis with a "membrane-like" feel, while also eliminating the fibrous properties of a warp-knitted structure. Moreover, the present invention allows the size of the trellis pores (e.g., pores 52 shown in Fig. 7) to be regulated. For example, the pore size (preferably about 100 microns or larger) can be regulated by controlling the quantity of resin applied to the exterior of the trellis. It is believed that regulation of pore size may facilitate assimilation of the trellis into the body.

In one preferred embodiment of the present invention, a medicinal substance (e.g., an antibiotic) is incorporated into the matrix encapsulating the yarns. The drug may be dispersed directly throughout the encapsulating resin or, alternatively, added to a plurality of separate carriers which in turn are dispersed throughout the encapsulating resin.

### EXAMPLES

The following examples illustrate the surgical support mesh of the present invention.

### EXAMPLE 1

A support trellis is woven of bi-component yarns, particularly 250 denier/16 filament/type LHCV Kanebo Bellcouple® polyester yarns. The Kanebo Bellcouple® yarn includes a polyethylene terephthalate polyester core and a polyethylene terephthalate/isophthalate co-polyester sheath, the sheath having a lower melting temperature than the core.

Following construction of the support trellis, the trellis is placed in a convection oven and heated to about 180°C, thus fusing the individual sheaths together. The yarns are thereby encapsulated and, further, are fused to each other at the junctions where the yarns overlap.

The resultant mesh is thereafter sealed in a sterile package.

### EXAMPLE 2

A support trellis is warp-knitted of bicomponent yarns, particularly 75 denier/24 filament/type LHCV Kanebo Bellcouple® polyester yarns. Following construction of the support trellis, the trellis is placed in a convection oven and heated to about 180°C, thus fusing the individual sheaths together.

The resultant mesh is thereafter sealed in a sterile package.

Although illustrative embodiments of the present invention have been described herein with reference to the accompanying drawings, it is to be understood that the invention is not limited to those precise embodiments, and that various other changes and modifications may be effected therein by one skilled in the art without departing from the scope or spirit of the invention.

The invention as described above can be summarized as follows:
A soft and pliable surgical support mesh exhibiting increased resistance to inhabitation of infectious matter. The mesh includes a support trellis formed of multifilament yarns wherein the interstitial voids located between the filaments of said yarns are enclosed within an infection-impervious matrix.

## Claims

1. A soft and pliable surgical mesh, comprising;
a support trellis formed of multifilament yarns encapsulated within an infection-impervious matrix whereby the interstitial voids located between the filaments of said yarns are enclosed within said matrix.

2. The mesh according to Claim 1, wherein said matrix exteriorly surrounds said yarns and is formed of a material having a flexibility at least as great as the flexibility of the material forming the filaments of said yarns.

3. The mesh according to Claim 2, wherein said matrix penetrates into said yarn to substantially fill the interstitial voids located therein.

4. The mesh according to Claim 3, wherein said matrix is formed from a polymer selected from the group consisting of polyester, polypropylene, polyethylene, polyurethane and copolymers thereof.

5. The mesh according to Claim 1, wherein said yarns are formed of a plurality of bi-component filaments each having a flexible core and a fusible sheath surrounding said core, and wherein said sheath has a fusing temperature lower than the melting temperature of said core whereby said sheaths fuse together to enclose said interstitial voids upon heating of said trellis.

6. The mesh according to Claim 5, wherein said core is formed of a polyethylene terephthalate polyester and said sheath is formed of a polyethylene terephthalate/isophthalate copolyester.

7. The mesh according to Claim 1, wherein said multifilament yarns overlap one another at regularly-spaced junctions to form said support trellis, and wherein said overlapping yarns are adhered to one another at said junctions to enhance ravel resistance of said mesh.

8. The mesh according to Claim 1, wherein said matrix is drug permeable, and wherein a medicinal drug is incorporated into said matrix for release into the body of a patient after implantation of said mesh therein.

9. A method of producing a soft and pliable surgical mesh exhibiting increased resistance to inhabitation of infectious matter from a support trellis formed of multifilament yarns, comprising:
encapsulating said multifilament yarns within an infection-impervious matrix whereby the interstitial voids located between the filaments of said yarns are enclosed with said matrix.
